(19) **Europäisches Patentamt / European Patent Office / Office européen des brevets**

(11) **EP 3 564 383 B1**

(12) **EUROPEAN PATENT SPECIFICATION**

(45) Date of publication and mention
of the grant of the patent:
**03.08.2022   Bulletin 2022/31**

(21) Application number: **17886404.7**

(22) Date of filing: **29.12.2017**

(51) International Patent Classification (IPC):
***C12P 19/02*** (2006.01)     ***C12P 19/24*** (2006.01)
***C12N 9/90*** (2006.01)

(52) Cooperative Patent Classification (CPC):
**C12P 19/02; C12N 9/90; C12P 19/24**

(86) International application number:
**PCT/KR2017/015780**

(87) International publication number:
**WO 2018/124833 (05.07.2018 Gazette 2018/27)**

(54) **METHOD FOR PRODUCING PSICOSE BY USING PSICOSE EPIMERASE PRODUCING MICROORGANISM**

VERFAHREN ZUR HERSTELLUNG VON PSICOSE UNTER VERWENDUNG VON PSICOSE-EPIMERASE-PRODUZIERENDEM MIKROORGANISMUS

PROCÉDÉ DE PRODUCTION DE PSICOSE À L'AIDE D'UN MICROORGANISME PRODUISANT UNE PSICOSE ÉPIMÉRASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **30.12.2016   KR 20160184093**

(43) Date of publication of application:
**06.11.2019   Bulletin 2019/45**

(73) Proprietor: **Samyang Corporation**
**Seoul 03129 (KR)**

(72) Inventors:
- **LEE, Jae Hoon**
  **Seoul 05507 (KR)**
- **KWON, Soun Gyu**
  **Gwangmyeong-si**
  **Gyeonggi-do 14311 (KR)**
- **PARK, Bu-Soo**
  **Hanam-si**
  **Gyeonggi-do 13014 (KR)**
- **PARK, Chong Jin**
  **Daejeon 34049 (KR)**
- **AHN, Sin Hye**
  **Goyang-si**
  **Gyeonggi-do 10374 (KR)**
- **LEE, Sang-Hee**
  **Daegu 41561 (KR)**
- **HAN, Eun Jin**
  **Seoul 05508 (KR)**

(74) Representative: **Schön, Christoph et al**
**Dr. Schön, Neymeyr & Partner mbB**
**Bavariaring 26**
**80336 München (DE)**

(56) References cited:
| | |
|---|---|
| EP-A1- 2 730 652 | EP-A1- 3 088 515 |
| WO-A1-2016/053035 | WO-A1-2016/201110 |
| KR-A- 20140 122 043 | KR-A- 20150 076 051 |
| KR-A- 20160 081 722 | US-A1- 2017 298 400 |

- KIM, N.-H.: "Conversion shift of D-fructose to D-psicose for enzyme- catalyzed epimerization by addition of borate", Applied and Environmental Microbiology, vol. 74, no. 10, May 2008 (2008-05), pages 3008-3013, XP055091184,
- PATEL, S. N.: "Improved operational stability of d-psicose 3-epimerase by a novel protein engineering strategy, and d-psicose production from fruit and vegetable residues", Bioresource Technology, 18 May 2016 (2016-05-18), pages 121-127, XP029631923, DOI: doi:10.1016/j.biortech.2016.05.053

**Description**

[TECHNICAL FIELD]

The present invention relates to a method for efficiently producing psicose using a microorganism producing psicose epimerase and a method for increasing psicose epimerase activity or expression of a microorganism expressing a psicose epimerase.

[BACKGROUND ART]

D-psicose (same name: psicose) is an isomerized sugar that a hydroxyl group at carbon 3 of fructose (D-fructose) is rotated by isomerization reaction, and a monosaccharide present in trace amounts in the natural world such as raisins, figs and wheat. In addition, D-psicose (same name: psicose) is an innovative sweetener that can solve the obesity caused by excessive intake of high-calorie saccharides in modern people, and the sweetness is a natural sweetness similar to sugar and the degree of sweetness is 70& compared to sugar [Oshima H et al, 2006]. In human body, the net energy gain of D-psicose (same name: psicose) is 0.007kcal/g (sugar 2.29 kcal/g, fructose 1.76 kcal/g), and it may be applied as a low-calorie sweetener of diet food, and it is a material capable of overcoming the phenomenon of deterioration in taste caused by reducing saccharides (Matsuo T et al, 2002).

In addition, it has a function of inhibiting the enzyme activity which involves in lipid synthesis in liver, and therefore the accumulation of abdominal fat can be inhibited, and thus it may be applicable to various functional foods such as health food, and so on, and it has a function of blood sugar inhibiting action by inhibiting absorption of glucose, and therefore it may be applied as a saccharide material for diabetes patients (Matsuo T et al, 2001, Hayashi N et al, 2010, Hossain A et al, 2015, Hossain A et al, 2015). The psicose having such sweetness properties and low-calorie, body fat accumulation inhibition, and saccharide absorption inhibition functionality is a material having the possibility to replace the broad market for which sugar accounts in the food industry, and it is a material which makes it possible to prevent diseases caused by obesity through healthy diet.

Currently, the development status of a microorganism for industrial production of psicose is represented by D-Psicose 3-epimerase recombinant enzyme from Agrobacterium tumefaciens (Korea, CJ), Arthrobacter globiformis (Japan, Matsutani), and currently, it is GRAS certified.

Since the psicose which has such potential economic value belongs to a rare sugar that is a monosaccharide extremely rarely present in the natural world, a method for producing it efficiently is needed to apply it to the food industry. As a biological catalyst has not been developed and commercialized, self-development is necessary. When using a recombinant enzyme for this, there is an advantage of being able to produce high efficiency of psicose, but there is a disadvantage of consuming a lot of cost and time to prove safety. On the other hand, when using a natural strain with proven safety (Non-GMO strain having edible experience), it is an important factor to reproduce the improved psicose production ability of the natural strain (Non-GMO strain having edible experience) in a high concentration culturing.

D-psicose-3-epimerase (DPEase), a representative psicose epimerase is very difficult to actually culture a microbial cell at a high concentration while maintaining high enzyme activity. Thus, for mass-production required for industrialization of psicose, there is a demand for a technology for culturing a microbial cell at a high concentration while maintaining DPEase maximum activity and a technology for producing psicose using thereof.

EP 2 730 652 A1 discloses a ketose 3-epimerase obtainable from a microorganism of the genus Arthrobacter, having substrate specificity to epimerize a D- or L-ketose at position 3 to produce a corresponding D- or L-ketose.

WO 2016/053035 A1 and its patent family member US 2017/298400 A1 disclose a method for producing psicose, comprising a step of reacting fructose as a substrate and an epimerase thereof in a microorganism at a temperature of 40°C or higher.

WO 2016/201110 A1 discloses the use of dihydroxyacetone (DHA) in the preparation of several natural and rare carbohydrates, for example psicose. Example 6 of WO 2016/201110 A1 discloses a method of producing D-psicose using psicose 3-epimerase from Agrobacterium Zumefaciens.

EP 3 088 515 A1 discloses a novel strain of Ensifer adhaerens isolated from soil and deposited under Accession No KCCM11405P as well as a method of producing psicose using said strain.

[DISCLOSURE]

[TECHNICAL PROBLEM]

An object of the present invention is to provide a method for increasing activity and/or expression of psicose epimerase of a microorganism having psicose conversion activity.

Another object of the present invention is to provide a method for producing psicose from fructose by culturing a microorganism capable of producing psicose epimerase.

[TECHNICAL SOLUTION]

The present invention provides a method for producing psicose as defined in claim 1.

In addition, a method for increasing psicose epimerase activity or expression of a microorganism expressing a psicose epimerase as defined in claim 6 is provided.

The method for producing psicose of the present invention comprises a step of preparing an initial culture medium; a step of culturing a microorganism producing psicose epimerase in the initial culture medium; and a step of converting fructose into psicose.

The microorganism applicable to the present invention is a microorganism capable of producing psicose epimerase (D-psicose-3-epimerase), and specifically, it relates to a microorganism producing psicose epimerase of which expression and/or activity is induced by psicose and/or fructose.

The microorganism is preferably a non-genetically modified microorganism comprising an inherent gene encoding psicose epimerase.

The non-genetically modified microorganism may comprise a wild type strain isolated from nature or a variant in which mutation is induced by various methods. The non-genetically modified microorganism is more suitable, since there is no problem of stability that a modified strain in which an exotic gene is introduced by gene manipulation has. In addition, a method for preparing the variant may be random mutation induced by external stimuli such as UV or NTG, for example, heat treatment.

The microorganism applicable to the present invention is one or more strains selected from the group consisting of Microbacterium foliorun, (M. foliorum). Microbacterium oxydans, Microbacterium phyllosphaerae and Ensifer adhaerens (E. adhaerens), and it is possible to use a microorganism for industrial purposes as a safety-proven strain including GRAS (Generally Recognized As Safe) strains. For example, M. foliorum strain of accession number KCCM11774P that is an improved strain selected and isolated from food, can be used as the Microbacterium foliorum strain in the methods of the present invention, and E. adhaerens strain of accession number KCCM11405P that is an improved strain selected and isolated from soil, can be used as the Ensifer adhaerens strain in the methods of the present invention.

Thus, the term used in the description of the present invention, "microorganism" means, one or more kinds selected from the group consisting of a microbial cell of the microorganism, a culture of the microorganism, a lysate of the microorganism, and supernatant of the lysate, and psicose epimerase may be comprised in one or more selected from the group consisting of a microbial cell of the microorganism, a culture of the microorganism, a lysate of the microorganism, and supernatant of the lysate. The culture comprises enzyme produced from the microorganism, and it may comprise the microorganism or be a cell-free form not comprising the microorganism. The lysate means a lysate lysing the microorganism or supernatant obtained by centrifuging the lysate, and it comprises enzyme produced from the microorganism.

Therefore, herein, a biocatalyst having psicose conversion activity means "microorganism", selected from the group consisting of a microbial cell of the microorganism, a culture of the microorganism, a lysate of the microorganism, and supernatant of the lysate, and it comprises one or more selected from the group consisting of a microbial cell of the microorganism, a culture of the microorganism, a lysate of the microorganism, and supernatant of the lysate, or psicose epimerase obtained therefrom.

In the present invention psicose is used as an inducer for increasing enzyme activity or expression of microorganism capable of producing psicose epimerase.

In the method for producing psicose according to the present invention, the microorganism is cultured in a medium further comprising a carbon source other than the inducer. The microorganism is cultured in a range of the inducer concentration (g/L) of 0.001 g/L to 5 g/L, and the microorganism is cultured under the condition of the pH variation value ($\Delta$pH) of the medium of 0.05 to 0.5. The inducer may be added to an initial culture medium in which the microorganism is inoculated, or an additional medium comprising the inducer may be added intermittently or continuously. In the additional medium, a ratio of carbon source to organic nitrogen source is 20/1 to 1/1. In addition, the microorganism may be cultured in a medium further comprising one or more of metal ions selected from the group consisting of manganese, cobalt, calcium, magnesium, nickel, iron and aluminum.

Herein, the inducer is 0.001 g/L to 5 g/L, 0.01 g/L to 5 g/L, 0.05 g/L to 5 g/L, 0.1 g/L to 5 g/L, 0.001 g/L to 3g/L, 0.01 g/L to 3 g/L, 0.05 g/L to 3 g/L, 0.1 g/L to 3 g/L, 0.001 to 1.5 g/L, 0.01 g/L to 1.5 g/L, 0.05 g/L to 1.5 g/L, or 0.1 g/L to 1.5 g/L.

The inducer used in the method of the present invention may be used as a carbon source alone, or is added together with other carbon source to a medium, for culturing the microorganism capable of producing psicose epimerase. In addition, the inducer used in the method of the present invention may be comprised in the initial culture medium used for inoculating and culturing the microorganism, an additional medium (feeding solution) supplied intermittently or continuously, or both the initial culture medium and additional medium.

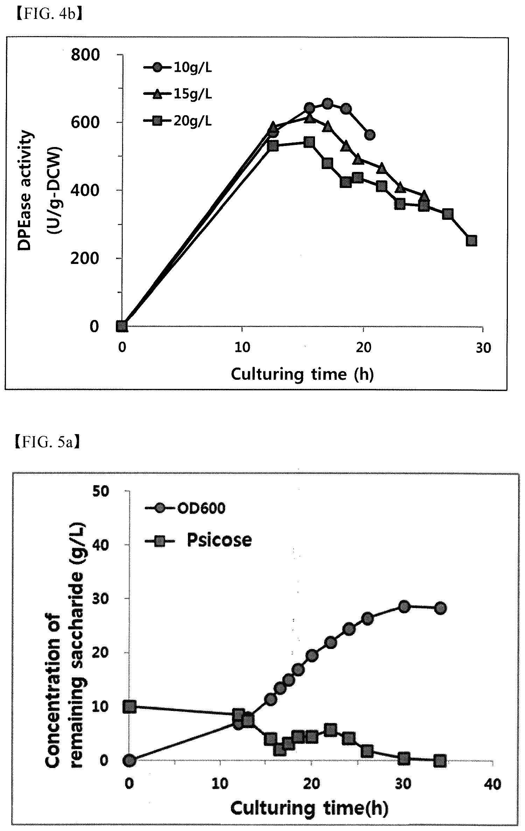

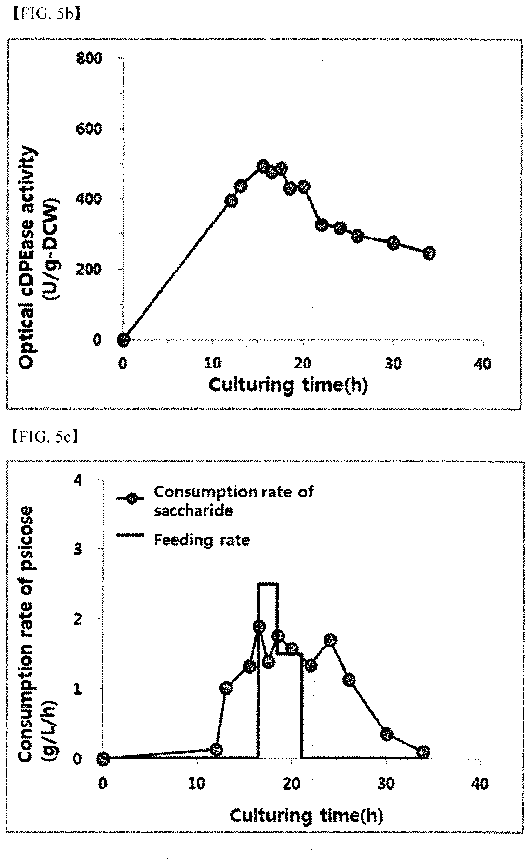

To produce psicose with high yield, when the psicose consumption rate in a medium is controlled artificially, the psicose consumption rate is increased as the psicose concentration in the medium is decreased due to DPEase activity increase, and therefore it is difficult to approach the actual psicose consumption rate, and accordingly, when sugar is additionally added in accordance with the psicose consumption rate artificially, sugar accumulation in the medium may be caused. The accumulated sugar may contribute to enhancement of microbial cell growth rate in proportion to the microbial cell yield to the sugar content, but it results in rapidly decreasing the activity of DPEase due to the increase of accumulated residual sugar (See FIG. 5a).

In addition, for mass-production of psicose, a high concentration culture of a microorganism for producing psicose is preferable, but the activity of psicose epimerase may be rapidly reduced as the high concentration microbial cell culture is progressed.

Thus, in order to increase enzyme activity and expression and psicose conversion activity and stably maintain psicose conversion activity during the culturing period, an inducer is added when culturing the microorganism for producing psicose, and also the inducer condition may be optimized, and selectively one or more of culturing conditions consisting of organic nitrogen source content, metal ion concentration and pH range may additionally be adjusted.

When Microbacterium foliorum, Microbacterium oxydans, Microbacterium phyllosphaerae or Ensifer adhaerens is used as the microorganism in the method of the present invention, psicose is used as an inducer, and fructose is used as additional other carbon source. When culturing with psicose, the contamination to other third microorganisms is inhibited, and the psicose yield per microbial cell is high, and the psicose epimerase activity is high.

The term used in the description of the present invention, "initial culture medium" or "initial medium" means a culture medium for inoculating and culturing a microorganism in the microorganism culturing.

The term used in the description of the present invention, "additional medium" means a medium additionally supplied intermittently or continuously to a culture medium after initiating culturing a microorganism other than the initial culture medium.

The term used in the description of the present invention, "culture" means a culture obtained by culturing a microorganism in the initial culture medium, additional medium or a mixed medium thereof.

Herein, the initial culture medium has a ratio of the content (g/L) of carbon source to organic nitrogen source, namely, C/N ratio of 10/2 to 10/10, or 10/3 to 10/10, 10/5 to 10/10. When supplying a carbon source and/or an organic nitrogen source to have the C/N ratio in the above range, DPEase having high expression and/or high activity may be induced and the enzyme may be obtained. Herein, for the term "ration of carbon source to nitrogen source" or "C/N ratio", the nitrogen source means an organic nitrogen source, more specifically, a complex organic nitrogen source, and the carbon source means a common carbon source used by a microorganism, but the carbon source may also perform the function of the inducer. For example, when batch culturing using the initial culture medium, in case that the sugar concentration of the initial medium is low, the enzyme activity is comparatively high at the late period of culturing when the carbon source (e.g. psicose) is almost consumed, but in case that the sugar concentration of the initial medium is low, the microbial cell concentration is too low and therefore the production yield per microbial cell should be considered, and thus the carbon source content range may be appropriately selected in consideration of enzyme activity and yield per unit microbial cell.

According to the present invention, a method for producing psicose, comprising a step of preparing an initial culture medium in which a ratio of carbon source to organic nitrogen source is 10/2 to 1/1; a step of culturing a microorganism capable of producing psicose epimerase in the initial culture medium; and a step of converting fructose into psicose, comprising reacting the cultured microorganism, a solution of the cultured microorganism, a lysate of the microorganism comprising psicose epimerase or a solution of the cultured microorganism with a fructose substrate.

When using a carbon source of the initial culture medium for producing the microorganism, for example, psicose, the psicose is a carbon source performing the function of the inducer, and it may be comprised so that the initial concentration of the carbon source is 3 g/L to 50 g/L, preferably, 5 g/L to 30 g/L, preferably, 5 g/L to 25g/L, more preferably, 5 g/L to 20g/L, 5 g/L to 15g/L, or 10 g/L to 20 g/L. In particular, the initial concentration of the carbon source, for example, the psicose in the culture medium does not largely affect the rate of increase of the microbial cell concentration to the culture time, but affects the maximum activity of DPEase, and therefore, when comprising a low concentration of psicose, relatively the highest DPEase activity may be obtained.

The carbon source used in the initial medium according to the the method of present invention, is one or more selected from the group consisting of psicose and fructose.

As a nitrogen source for effective culturing of a microorganism used for a method for producing psicose of the present invention, an organic nitrogen source is comprised, and additionally an inorganic nitrogen source may be comprised. The organic nitrogen source may be one or more kinds of organic nitrogen compounds from the group consisting of yeast extract, corn steep liquor, soytone, peptone, soybean, soybean meal, cottonseed meal, molasses, casein, beef extract, malt extract and tryptone, and preferably, yeast extract may be used.

The microorganism culturing according to the present invention is performed so that a pH variation value of the medium is 0.05 to 0.5. The microorganism culturing is performed so that the carbon source concentration (g/L) of the medium is maintained at 0.001 g/L to 5 g/L, and it is performed by supplying an additional medium comprising the inducer intermittently or continuously.

The microorganism culturing of the present invention is performed by one or more kinds of methods selected from the group consisting of batch culturing, fed-batch culturing and continuous culturing, and preferably, one or more methods selected from the group consisting of fed-batch culturing and continuous culturing may be used so as to maintain the carbon source of the culture solution at a low concentration. The fed-batch culturing in which a user sets the injection rate of sugar according to microorganism growth pattern analysis and sugar is injected may lead to the enhancement of productivity and yield compared to batch culturing. The fed-batch culturing is a method of batch culturing by setting an appropriate sugar concentration initially and gradually injecting a high concentration of sugar to obtain products at a concentration desired by a user, to maintain sugar in the culture solution at a low concentration. Then, according to addition time, addition method and added amount of sugar, various fed-batch culturing methods are present, and they may affect the growth rate of microorganism or production yield and enzyme activity of intracellular enzyme, and so on according to each condition. For example, herein the microorganism culturing by fed-batch culturing or continuous culturing may be performed by a pH stat feeding method.

The pH stat feeding method is known to have a small change in saccharide concentration and to match the growth tendency of microorganisms, and therefore it has been widely used for culturing aerobic microorganisms. By noting that DO (dissolved oxygen) is increased when the carbon source depletes, DO-stat has an advantage that cells can be automatically at a high concentration by injecting carbon sources when DO is increased, but since a DO probe sensor is expensive than a pH sensor and its maintenance is difficult, pH stat is more preferable for industrial use in culturing for mass-production.

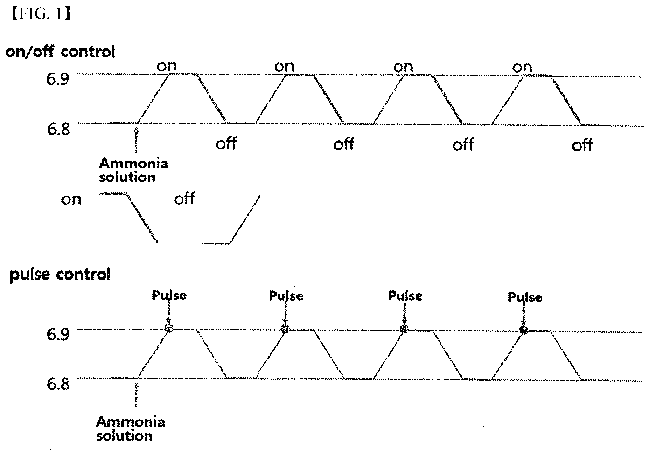

Thus, the pH-stat fed-batch culturing method is to utilize a pH drop phenomenon occurred as a microorganism consumes saccharides and metabolizes, and an object is to inject saccharides in a range of maintaining the minimum saccharide concentration and not lowering the activity of the microorganism. This is a method in which a small amount of saccharides are injected when a desired pH is set and a pH is increased finely, and when a small amount of saccharides are metabolized, the pH is finely decreased, and when all the saccharides are consumed, the pH is increased again, thereby repeating a cycle in which the saccharides are injected again. However, the enzyme production through the conventional pH-stat method is an on/off control method, and it is continuously supplied until the pH value is lowered when it is supplied once and a change in the supply amount is caused after a certain time, and the enzyme activity may be decreased. Therefore, preferably, the culturing method used for the method for producing of the present invention may use a method for controlling a cell growth rate in a certain substrate supply amount through a pulse method capable of controlling in a certain amount not the on/off control method, when pH increase and fall occur as fermentation proceeds (FIG. 1).

The present invention may supply a carbon source so that the pH of the initial culture medium or culture solution is pH 6.0 to 7.5, preferably, pH 6.5 to 7.5, more preferably pH 6.5 to 7.0 and the pH is maintained in the above pH range. When the pH is maintained, the DPEase activity may be maintained high without saccharide accumulation.

In the microorganism culturing of the present invention, when a carbon source is supplied by the method of maintaining the pH range, a pH variation value or difference value in the culture solution is 0.5 or less, 0.4 or less, 0.3 or less, 0.2 or less, or 0.1 or less, and it is 0.05 or more. For example, so that the pH variation value in the culturing solution is maintained at 0.05 to 0.5, preferably 0.05 to 0.15, an additional medium comprising a carbon source may be supplied. In the pH stat feeding method, a basic solution such as sodium hydroxide or ammonia solution is supplied so that it is not decreased to a certain pH or less. Then, when the pH variation value is smaller than 0.05, the pH rise width due to basic solution supply may be temporarily increased than 0.05, and this is recognized as a pH increase by saccharide depletion and an excessive amount of saccharide solution is added and thereby saccharide accumulation is caused, and thus it may cause DPEase activity reduction. On the other hand, when the pH variation value is bigger than 0.5, the state in which saccharide is depleted is prolonged and therefore a microbial cell growth rate is delayed, and thus the DPEase activity may be inhibited. Thus, when the pH is maintained, the enzyme activity may be maximized while minimizing the saccharide accumulation time.

In the culturing step of the microorganism of the present invention, by maintaining the concentration of the carbon source in a culture at a concentration in a range of 0.001 g/L to 5 g/L, 0.01 g/L to 5 g/L, 0.05 to g/L 5 g/L, 0.1 g/L to 5 g/L, 0 g/L to 3 g/L, 0.001 g/L to 3 g/L, 0.01 g/L to 3 g/L, 0.05 g/L to 3 g/L, 0.1 g/L to 3 g/L, 0 g/L to 1.5 g/L, 0.001 g/L to 1.5 g/L, 0.01 g/L to 1.5 g/L, 0.05 g/L to 1.5 g/L, 0.1 g/L to 1.5 g/L, preferably 0.1 g/L to 1 g/L, the DPEase activity may be maintained high without saccharide accumulation, and preferably, the concentration of the carbon source in the culture solution may be maintained in the range by the pH stat feeding method. In the fed-batch and continuous culturing, it is preferable to achieve the concentration of the carbon source in the above numerical range.

In the microorganism culturing of the present invention, an f value defined as the carbon source concentration (g/L) increased in the culture solution when the carbon source (for example, saccharides) is supplied once in an additional medium form as the following equation 1, namely, the increase of the carbon source concentration (g/L) in the culture solution when an additional medium comprising the carbon source is supplied once, is 0.2 to 0.5, preferably 0.25 to 0.3.

[Equation 1]

$$f = \text{(carbon source concentration in culture solution right after supplying additional medium)} - \text{(carbon source concentration in culture solution right before supplying additional medium)}$$

When the f value is smaller than 0.5, it is possible to prevent being an intermittent feeding method by reducing the accumulation time of the carbon source, and the DPEase activity may be maintained high. In addition, since the increasing pH rate as the f value is maintained at 0.25 or more and it enters the high concentration culture is slower than the decreasing pH rate by adding the carbon source, it is possible to prevent deviating from the pH-stat section.

When using yeast extract as a nitrogen source in an additional medium, the yeast extract may be comprised in the additional supply medium at a concentration of 10 g/L to 400 g/L, preferably 20 g/L to 200 g/L, more preferably 60 g/L to 100 g/L. By comprising the yeast extract in the above range, regardless of the microbial cell concentration, the activity of the psicose epimerase, DPEase may be stably maintained.

The ratio of carbon source to organic nitrogen source of the additional medium of the present invention, namely, C/N ratio is 20/1 to /1/1, preferably 10/1 to 2/1, more preferably 10/1 to 3/1, and by supplying a carbon source and/or nitrogen source having a C/N ratio in the above range, the activity of DPEase may be maintained at maximum even in the high concentration of microbial cell. The carbon source and nitrogen source are as described in the carbon source and nitrogen source of the initial culture medium.

The initial culture medium or additional medium of the method for producing psicose of the present invention may further comprise a metal ion. The metal ion may be added to the carbon source, or may be added to the mixture of the microorganism and the carbon source. It may also be added to a carrier in which a microorganism producing DPEase is immobilized (before adding the carbon source), or may be added to the mixture of the carrier in which the microorganism is immobilized and the carbon source (after adding the carbon source), or may be added in a form of the mixture with the carbon source when adding the carbon source or separately.

The metal ion may be one or more kinds selected from the group consisting of manganese ion, calcium ion, magnesium ion, nickel ion, cobalt ion, iron ion and aluminum ion, and so on. The metal ion may act as a coenzyme which increases the activity of enzyme by increasing the binding capacity of fructose and DPEase. Preferably, cobalt and/or manganese ion may be used, and copper and zinc may cause reduction of activity. Cobalt is not allowed as a food additive, but manganese has been approved as a food additive.

When the metal ion of the present invention is supplied as comprised in an additional medium, it may be comprised at a concentration of 0.1 mM to 5 mM, 0.1 mM to 4 mM, 0.1 mM to 3 mM, preferably 0.5 mM to 2.5 mM, in the additional supply medium, and the difference of the microbial cell growth rate according to the metal ion is insignificant, but when comprised at a low concentration or high concentration, the DPEase activity is highest, and therefore psicose may be produced with excellent yield.

The psicose conversion enzyme (DPEase) or microbial cell of the microorganism producing psicose conversion enzyme used in the present invention may be filled in a column for immobilization reaction as comprised in a carrier. Then, it is very important industrially to consistently maintain the activity of psicose conversion enzyme high in the mass-production process of psicose, since there are effects that the productivity is increased and also the production rate becomes fast when the activity of psicose conversion enzyme is maintained high stably.

The carrier used herein can create an environment in which the activity of the immobilized strain, or the enzyme produced from the strain is maintained for a long period, and it may be all carriers capable of being used for enzyme immobilization known in the art. For example, as the carrier, sodium alginate may be used. Sodium alginate is a natural colloidal polysaccharide present rich in cell walls of seaweed, and is composed of mannuronic acid (β-D-mannuronic acid) and gluronic acid (a-L-gluronic acid), and in terms of the content, is formed randomly by creating beta-1,4-bonds, and the strain or enzyme is stably immobilized, and therefore it may be advantageous to exhibit excellent psicose yield.

To enhance the yield of psicose more, sodium alginate solution (for example, sodium alginate aqueous solution) may be used for immobilization of a strain. For example, by dropping the obtained mixed solution to about 0.2M calcium ion solution using an injection pump and a vacuum pump, after adding and mixing a microbial cell of the strain, a culture comprising enzyme produced by the strain, or lysate of the strain to sodium alginate aqueous solution of 1 to 2 times by volume of a microbial cell of the strain, a culture comprising enzyme produced by the strain, or lysate of the strain, thereby generating beads, a microbial cell of the strain, a culture comprising enzyme produced by the strain, or lysate of the strain may be immobilized. The enzyme may be purified from the strain, strain culture or lysate of the strain by common methods, for example, methods such as dialysis, precipitation, adsorption, electrophoresis, affinity chromatography, ion exchange chromatography, and so on.

An additional example of the present invention provides a method for producing psicose using a fructose-containing substrate using a bead comprising the enzyme or microbial cell and a carrier.

Preferably, the method for producing psicose of the present invention may be performed by filling the bead containing the enzyme or microbial cell to a column and flowing the fructose-containing substrate solution, and it may be performed by easily selecting suitable one according to the used enzyme or microbial cell, or immobilization carrier by those skilled in the art.

When fructose solution is supplied to a filling column in which a microbial cell comprising psicose epimerase is filled at a certain concentration, the epimerization reaction is carried out by the immobilized microbial cell, and thereby fructose is converted into psicose. The converted psicose may be produced in a psicose syrup (liquid) or powder (solid) form after isolation and purification using a separation column, and so on.

[ADVANTAGEOUS EFFECTS]

The present invention is a method for producing psicose with high yield using a microorganism, selected from Microbacterium foliorum, Microbacterium oxydans, Microbacterium phyllosphaerae and Ensifer adhaerens, which produces DPEase, and it can culture the microorganism at a high concentration and also stably maintain the enzyme activity and produce psicose with high yield, and thus it is expected to be widely used in the functional sugar-related health functional food and drug industries.

[BRIEF DESCRIPTION OF DRAWINGS]

FIG. 1 schematizes the method for culturing according to the on/off control method and pulse control method described in the description of the present invention.

FIG. 2a shows the optical microbial cell concentration and DPEase activity of Ensifer sp. strain according to the kind of carbon sources.

FIG. 2b shows the optical microbial cell concentration and DPEase activity of Microbacterium sp. strain according to the kind of carbon sources.

FIG. 3a shows an optical microbial cell concentration in batch culturing using psicose and fructose as carbon sources.

FIG. 3b shows a remaining sugar concentration in batch culturing using psicose and fructose as carbon sources.

FIG. 3c shows DPEase activity according to the culturing time in batch culturing using psicose and fructose as carbon sources.

FIG. 4a shows an optical microbial cell concentration according to the initial concentration of psicose (10, 15 and 20 g/L).

FIG. 4b shows DPEase activity according to the initial concentration of psicose (10, 15 and 20 g/L).

FIG. 5a shows an optical microbial cell concentration and a remaining sugar concentration when culturing in an intermittent sugar supply method.

FIG. 5b shows DPEase activity when culturing in an intermittent sugar supply method.

FIG. 5c shows a psicose consumption rate when culturing in an intermittent sugar supply method.

FIG. 6a shows an optical microbial cell concentration and a remaining sugar concentration when culturing in a pH stat sugar supply method.

FIG. 6b shows DPEase activity when culturing in a pH stat sugar supply method.

FIG. 7a shows an optical microbial cell concentration according to the yeast extract concentration (20, 40 and 80g/L).

FIG. 7b shows DPEase activity according to the yeast extract concentration (20, 40 and 80g/L).

FIG. 8a shows an optical microbial cell concentration according to the manganese ion concentration (0.5, 1 and 2mM).

FIG. 8b shows DPEase activity according to the manganese ion concentration (0.5, 1 and 2mM).

[MODE FOR INVENTION]

The present invention will be described in more detail by the following exemplary examples.

**Example 1: Effect of kinds of medium carbon source on psicose epimerase**

1.1 Used microorganism

In culturing of a mutant strain with enhanced psicose productivity of which gene is not artificially manipulated, a medium carbon source may affect the activity of DPEase that is psicose epimerase. The microorganism used in the present example is a mutant strain improved by accelerating natural evolution, and two kinds of mutant strains, Microbacterium foliorum (M. foliorum) and Ensifer adhaerens (E. adhaerens) were used.

The E. adhaerens mutant strain is a gram negative bacillus isolated from rhizosphere soil, and contains DPEase that is psicose epimerase, and is E. adhaerens SYG29 strain of accession number KCCM11405P disclosed in Korean Publication Patent No. 10-2014-0122043 that is an improved strain derived from soil which is selected and isolated.

In addition, the M. foliorum strain is non-pathogenic, and is a gram positive micrococcus classified to biosafety degree 1 that does not form a spore, and contains DPEase that is psicose epimerase and is an improved strain derived from food which is selected and isolated, and it was isolated by using the following method.

1% (w/v) psicose-added Mineral salt broth (KH2PO4 2.4 g/L, K2HPO4 5.6 g/L, (NH4)2•SO4 2.6 g/L, MgSO4•7H2O 0.1 g/L, yeast extract 1 g/L) was used. Food (for example, broccoli, ginseng, edible flowers, etc.) was selected and 1g of each food was collected and added to MSP broth, and then cultured at 30°C for 24 hours, thereby conducting enrichment. Then, 100 μL (microliter) of culture solution was collected and painted over an agar medium, and then was cultured at 30°C until colonies were confirmed. After selecting colonies which were different in shape and size among colonies formed in the agar medium and inoculating MSP broth, it was shaking cultured at 30°C for 24 hours and centrifuged, thereby collecting microbial cells only. The collected microbial cells were added to 100 μL of 50 mM PIPES (piperazine-N, N'-bis(2-ethanesulfonic acid)) buffer solution (pH 7.0) and suspended, and lysed using an ultrasonic processor (ColepParmer) to obtain lysate solution. After the lysate solution was centrifuged at 12,000rpm at 4°C for 10 minutes, the supernatant was collected and used as crude enzyme, and the crude enzyme was reacted at 30°C for 12 hours using 10 mM fructose and psicose as substrates.

It was confirmed that whether psicose was converted into fructose in the reaction solution through thin layer chromatography (TLC) analysis. The thin layer chromatography analysis was performed by developing for 10 minutes three times using a silica gel (Silica gel 60F254(Merck, Germany))having a width of 20cm and a length of 10cm,fixed bed and a mobile bed developing solvent in which acetonitrile and water was mixed at a volume ratio of 85:15.

The strain in which the conversion from psicose into fructose was confirmed through the TLC analysis was selected and inoculated to 0.1%(w/v) psicose-added MS broth, and shaking cultured at 30°C for 24 hours, and after centrifugation, only microbial cells were collected. The collected microbial cells were washed with 0.85%(w/v) NaCl and then added to 50 mM PIPES buffer solution (pH 7.0) in which 400 g/L fructose and 1 mM manganese ion were added and suspended, and reacted at 70°C for 1 hour.

Then, the reacted products were centrifuged and the supernatant was collected, and then high-performance liquid chromatography (HPLC) analysis was carried out. The liquid chromatography analysis was performed using RID (Refractive Index Detector, Agilent 1260 RID) of HPLC (Agilent, USA) equipped with Aminex HPX-87C column (BIO-RAD). As the mobile bed solvent, water was used, and the temperature was 80°C and the flow rate was 0.6 mL/min. The obtained result was shown in FIG. 1, and one strain which produced psicose the most, M. foliorum was finally selected among 1500 kinds of strains, and it was named Microbacterium foliorum SYG27B, and was deposited to Korean Culture Center of Microorganisms on September 24, 2015 and received Accession number KCCM11774P.

## 1.2 Culture conditions

Using monosaccharides, psicose, fructose, glucose, galactose and mannose as the medium carbon source of the strain of Example 1.1, according to the seed culture medium composition of the following Table 1, a medium was mixed, and for death of all strains, by treating high temperature and high pressure steam sterilization at the temperature of 121°C for 15 minutes, each seed medium was prepared. The culture was carried out at the temperature of 30°C and 240 rpm according to flask culturing method.

[Table 1]

| Medium source | Primary seed culture |
|---|---|
| Carbon source (g/L) | 5 |
| $MgSO_4$-$7H_2O$ (g/L) | 0.5 |
| Yeast extract (g/L) | 2 |
| $(NH_4)_2SO_4$ (g/L) | 7 |
| $KH_2PO_4$ (g/L) | 0.5 |
| $K_2HPO_4$ (g/L) | 0.5 |
| $FeSO_4$-$7H_2O$ (mg/L) | 6 |
| $MnSO_4$-$4H_2O$ (mg/L) | 4 |

(continued)

| Medium source | Primary seed culture |
|---|---|
| Biotin (mg/L) | 0.2 |
| Thiamine-HCl (mg/L) | 0.2 |
| Nicotinamide (mg/L) | - |
| Antifoaming agent (mL) | - |

1.3 Enzyme activity measurement

In order to measure the psicose epimerase activity of the cultured strain in Example 1. 2, after collecting 0.1ml to 1ml of culture solution, microbial cells and culture solution were separated by centrifugation and the culture supernatant was removed to collect microbial cells. Based on the optical microbial cell density of each sample (OD, 600nm), the dry microbial cell mass was calculated, and based on the dry microbial cell mass vs substrate solution volume ratio of 2.5 mg/ml, the high concentration fructose substrate solution volume to be added compared to the dry microbial cell mass was calculated and added. Herein the high concentration fructose substrate solution means a solution in which 400 g/L fructose and 1 mM manganese metal ion were added using 50 mM PIPES buffer solution (pH 7.0) as a solvent.

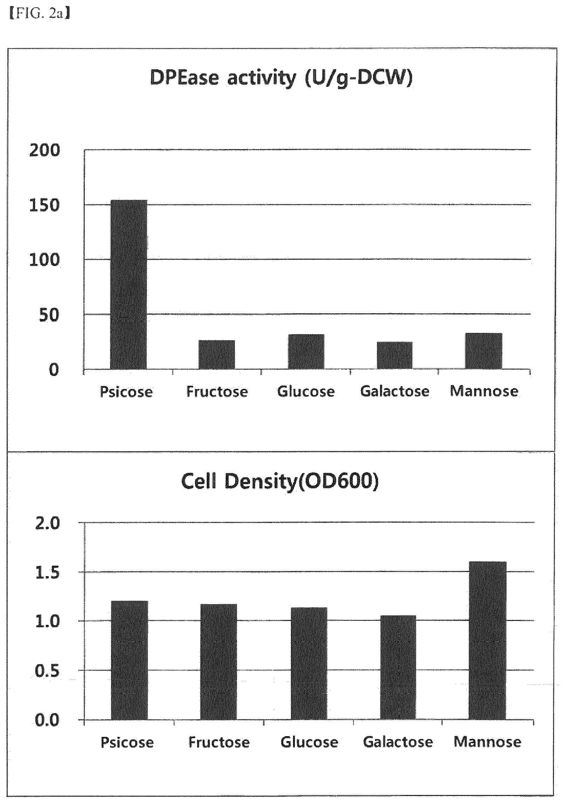

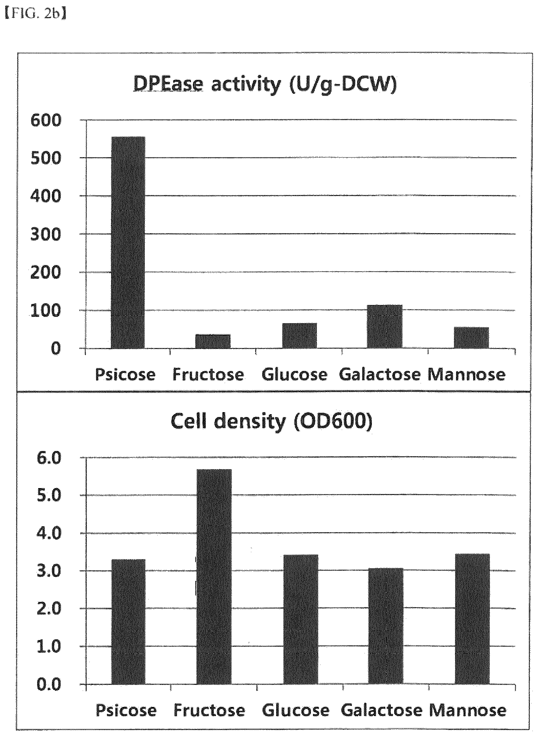

After each sample was fixed at the temperature of 70°C for one hour under the same condition, the microbial cells and reaction supernatant were separated through centrifugation to terminate the reaction. For HPLC analysis, after diluting the reaction supernatant with distilled water at the solid content less than 2%, it was passed through a 0.2um filter and analyzed. HPLC analysis was progressed using BIORAD Aminex HPX-87C column (mobile bed D.W, flow rate 0.6mL/min, temperature 90°C, RT 40 minutes), and psicose and fructose standard solution was prepared and each concentration to the HPLC area was qualified by writing standard calibration curves. Through the analysis of concentration of psicose produced from fructose per unit time, the degree of enzyme activity of each sample was compared and the result was illustrated in FIG. 2a and FIG. 2b.

The enzyme activity means the psicose conversion rate per unit DCW microbial cell reacted at 80°C for one hour using microbial cells of 2mg DCW (Unit/g-DCW).

In view of the microbial cell concentration and enzyme activity of Ensifer sp. strain shown in FIG. 2a and Microbacterium sp. strain shown in FIG. 2b, in particular, Microbacterium sp. strain had the highest microbial concentration when culturing with fructose, and the Ensifer sp. strain had it when culturing with mannose, and they showed similar microbial cell concentrations for other monosaccharide carbon sources. However, in case of DPEase activity, both strains showed relatively very high activity when culturing with psicose. This means that psicose directly affects the DPEase high activity expression. It was confirmed that the microbial cell growth rate and activity of Microbacterium sp. strain were higher than Ensifer sp. strain, and the following example was progressed using Microbacterium sp. strain.

**Example 2: Batch culture of the microorganism using fructose and psicose**

In culturing of M. foliorum, when using fructose or psicose as a carbon source, compared to other carbon source, as same as the result of Example 1, the high microbial concentration or high DPEase activity can be induced. Since the bilateral reaction enzyme, DPEase can directly use fructose and psicose as a substrate, when using fructose or psicose as a medium carbon source, it may be effective for high activity DPEase expression. Thus, when using fructose and psicose which were DPEase substrates as medium carbon sources, the high activity DPEase expression and microbial cell growth were compared.

For high concentration culture of M. foliorum, each seed medium was prepared with the composition of the Table 1, and then each seed medium was prepared by treating high temperature and high pressure steam sterilization at the temperature of 121°C for more than 15 minutes. The composition of each seed medium and main culture medium (initial culture medium) was selected as the following Table 2 in consideration of price competitiveness and productivity, and to increase the DPEase activity, DPEase was induced with fructose and psicose.

[Table 2]

| Medium source | Primary seed culture | Secondary seed culture | Main culture |
|---|---|---|---|
| Psicose or fructose (g/L) | 5 | 5 | 10 |
| MgSO₄-7H₂O (g/L) | 0.5 | 0.5 | 1.5 |
| Yeast extract(g/L) | 2 | 2 | 4 |

(continued)

| Medium source | Primary seed culture | Secondary seed culture | Main culture |
|---|---|---|---|
| $(NH4)_2SO_4$ (g/L) | 7 | 7 | 7 |
| $KH_2PO_4$ (g/L) | 0.5 | 0.5 | 0.5 |
| $K_2HPO_4$ (g/L) | 0.5 | 0.5 | 0.5 |
| $FeSO_4$-$7H_2O$ (mg/L) | 6 | 6 | 12 |
| $MnSO_4$-$4H_2O$ (mg/L) | 4 | 4 | 8 |
| Biotin (mg/L) | 0.2 | 0.2 | 0.2 |
| Thiamine-HCl (mg/L) | 0.2 | 0.2 | 0.2 |
| Nicotinamide (mg/L) | - | - | 10 |
| Antifoaming agent (mL) | - | - | 0.5 |

For seed preparation, M. foliorum mother stored at the temperature of -70°C was inoculated in a 3ml seed medium and was under primary seed culture at the temperature of 30°C for 24 hours, and then was inoculated in a 100ml seed medium and the secondary seed culture was progressed at 30°C for 24 hours. The secondary seed medium was finally inoculated in a 2L main culture producing medium using a 5L fermenter and cultured at 30°C. The air supplied into the fermenter was used as antibacterialized using a 0.2um air filter, and the culture was carried out according to the fermenter culture condition of Table 3.

[Table 3]

| Culture step | Primary seed culture | Secondary seed culture | Main culture |
|---|---|---|---|
| Culture equipment | Test tube | Flask | 5L fermenter |
| Culture volume | 0.003L | 0.1L | 2L |
| Inoculation volume | 3% (v/v) | 6% (v/v) | 5% (v/v) |
| Culture time | 24hr | 24hr | 30-40hr |
| RPM | 200 | 200 | 500-800 |
| Air (vvm) | - | - | 2 |
| * vvm (volume of air per volume of liquid per minute): L/min <br> * Culture temperature: 30°C | | | |

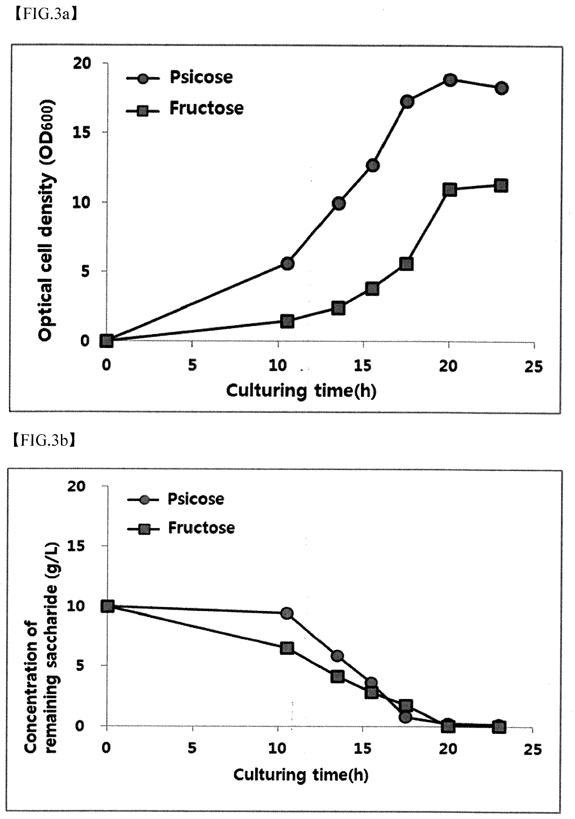

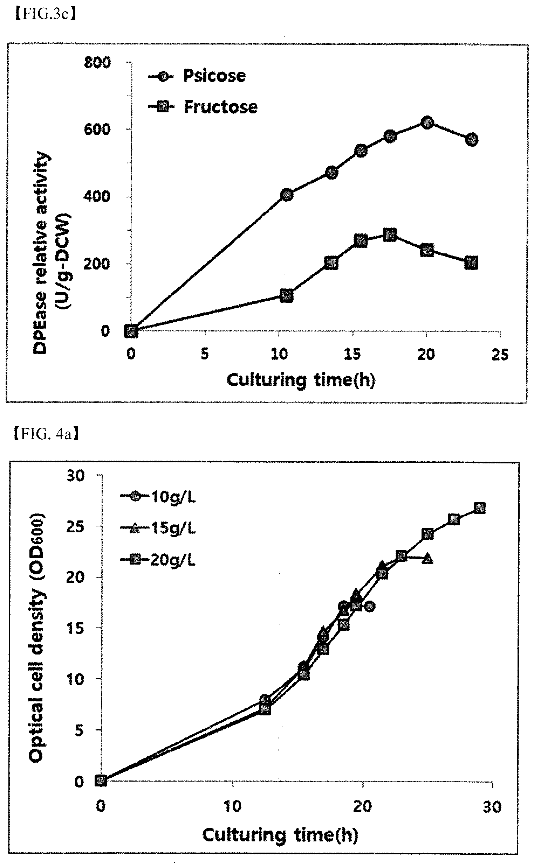

With the method of Example 1.3, through fructose and psicose content analysis, the degree of enzyme activity of each sample was compared and the result was illustrated in FIG. 3a to FIG. 3c.

As shown in FIG. 3a, comparing the corresponding microbial cell yield in batch culture, psicose was OD600 2.88/ (1g/L psicose) and fructose was OD600 1.13/(1g/L fructose), and psicose could produce microbial cells effectively 2.5 times using the same concentration of saccharides, and therefore culturing with psicose was more economic. In addition, considering the remaining saccharide analysis result, psicose was reduced while showing the tendency of log function, and fructose was reduced while showing the tendency of linear function (FIG. 3b). This tendency means the result occurred as psicose is not easily ingested in microbial cells compared to fructose, and means that the saccharide is hardly ingested during inducing the high activity DPEase expression for survival of microbial cells and as the DPEase activity is enhanced at a certain level, the psicose consumption rate is increased. Thus, since psicose can induce the high activity DPEase expression effectively than fructose, the following example was carried out using psicose as a carbon source.

**Example 3: Batch culture of the microorganism according to psicose initial concentration**

In order to confirm the concentration of carbon source in the optimum psicose initial culture medium, the initial psicose concentration was selected to 10g/L, 15g/L and 20g/L, and fermentation was progressed as Example 2. In addition, as same as the measurement method of activity conducted in Example 1.3, the bioconversion reaction of fructose was progressed, and the result obtained by measuring psicose production by HPLC and comparing the activity was shown in FIG. 4a and FIG. 4b.

As shown in FIG. 4a and FIG. 4b, it was confirmed that regardless of the initial psicose concentration, the rate of increase of microbial cell concentration to the culture time was similar, and it was confirmed that the maximum DPEase activity was raised as the initial psicose concentration was low. Thus, 10g/L initial psicose concentration was selected as the optimum concentration, and the following example was carried out.

**Example 4: Fed-batch high concentration culture of the microorganism**

4-1. Analysis of activity according to the intermittent saccharide supply method

In order to confirm the effectiveness of the intermittent saccharide supply method, under the condition that saccharides were supplied every fixed time at a rate of 1.5-2.5g/L/h, fermentation was progressed as Example 2. In addition, as same as the measurement method of activity conducted in Example 1.3, the bioconversion reaction of fructose was progressed, and the result obtained by measuring psicose production by HPLC and comparing the activity was shown in FIG. 5a to FIG. 5c.

As shown in FIG. 5c, when the psicose consumption rate was controlled artificially, the psicose consumption rate is increased as the psicose concentration in the medium was reduced due to the increase of DPEase activity, and therefore it is difficult to approach the actual psicose consumption rate, and this may cause saccharide accumulation as FIG. 5a. The accumulated saccharides might contribute to the microbial cell growth rate improvement in proportion to the microbial cell yield to the saccharide content, but it caused dramatical reduction of the activity of DPEase due to the increase of accumulated residual saccharides.

4.2 Analysis of activity according to pH-stat method

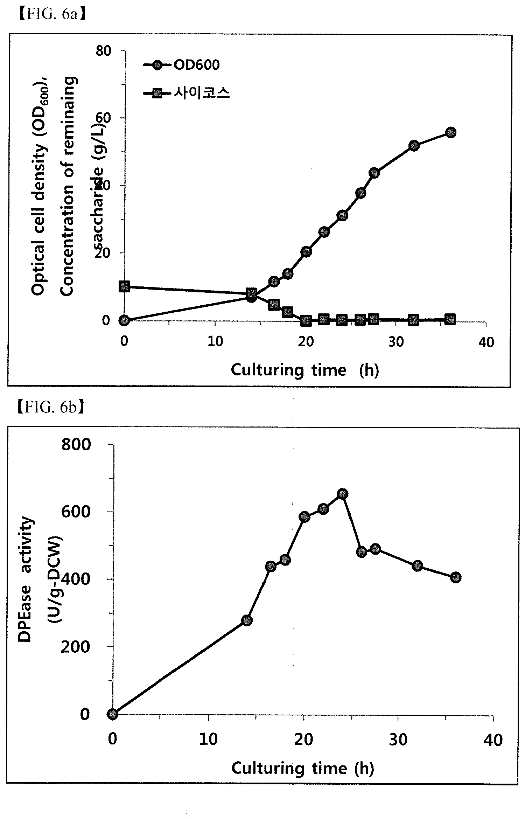

In order to confirm the effectiveness of the pH-stat saccharide supply method, fermentation was progressed as Example 2. The increase of the carbon source concentration (g/L) in the culture solution (fermenter) when the carbon source was added once in the form of the additional medium was define as the f value, and the time of adding the carbon source was adjusted so that the f value was 0.25. The composition of the additional medium, namely feeding solution was psicose 400g/L, yeast extract 40g/L and $MnCl_2$ 1mM. According to the pH-stat fundamental principle, when pH was over 6.95, saccharides were supplied and thereby pH was reduced, and the pH was controlled through 9% ammonia water and sugar solution in the pH range of 6.8-6.95. In addition, as same as the measurement method conducted in Example 1.3, the activity was measured, and the result obtained by comparing the activity was shown in FIG. 6a, FIG. 6b and Table 4. In Table 4, the microbial cell mass (DCW) can be obtained by applying $OD_{600}$ value to Equation 2, as $OD_{600}$ value 1 corresponds to 0.35 DCW/L when the microbial cell concentration is represented by $OD_{600}$ value (g-DCW/L)=0.35 × $OD_{600}$).

[Equation 2]

$$DCW(g)/L = 0.35 \ (g/L) \ X \ OD \ value$$

[Table 4]

| Culture time (hr) | Microbial cell concentration ($OD_{600}$) | Microbial cell mass (DCW) | Enzyme activity (U/g-DCW) |
|---|---|---|---|
| 14 | 7 | 2.45 | 286 |
| 16.5 | 11.62 | 4.07 | 447 |
| 18 | 13.91 | 4.87 | 460 |
| 20 | 20.54 | 7.19 | 584 |
| 22 | 26.34 | 9.23 | 616 |
| 24 | 31.3 | 10.96 | 671 |
| 26 | 37.93 | 13.28 | 489 |
| 27.5 | 43.88 | 15.36 | 501 |

(continued)

| Culture time (hr) | Microbial cell concentration (OD$_{600}$) | Microbial cell mass (DCW) | Enzyme activity (U/g-DCW) |
|---|---|---|---|
| 32 | 52 | 18.2 | 446 |
| 36 | 56 | 19.6 | 413 |

As shown in FIG. 6a, the pH-stat was progressed 18 hours after the batch culture, and different from the intermittent feeding method, the saccharide concentration was consistently maintained between the range of 0g/L to 1g/L. In addition, compared to the maximum activity of the microbial cell cultured by the intermittent feeding method of approximately 500U/g, the maximum activity of the microbial cell cultured by the pH-stat method was approximately 650U/g and was increased 1.3 times. This means that when a trace of psicose is present, the activity of psicose epimerase may be increased.

However, the result that the activity of the psicose epimerase was rapidly reduced as the high microbial cell culture of OD$_{600}$ 30 or more was progressed was caused. This is the result occurred in the pH-stat process, and it was confirmed that it could be solved by adjusting the f value, pH value and composition of feeding solution.

When the f value is too large, the time when the carbon source, namely, saccharide is accumulated is extended and finally it becomes close to the intermittent feeding method, and it may cause the reduction of DPEase activity. On the other hand, when the f value is too small, as the rate of reducing pH by adding the carbon source is slow than the rate of increasing pH according to the entry of high concentration culture, it may escape the pH-stat range, and the control of pH-stat may be impossible. As can be seen in FIG. 6a, as the saccharide was not accumulated and was maintained at a certain level, and it did not escape the pH-stat range and was stably progressed, it is difficult to see that the f value was a big problem. It was confirmed that as the pH value was low, the high activity of DPEase could be obtained, but it was necessary to have the pH upper limit value higher than the ammonia water added, and therefore the minimum value in the present fermenter, ΔpH=0.15 was preferable. Thus, the other variable, the composition of the feeding solution was controlled to maintain the high activity in the high concentration culture.

**Example 5: Establishment of conditions for maintaining activity according to additional medium composition**

As mentioned in Example 4, by controlling the composition of the additional medium (feeding solution), the high concentration strain culture conditions expressing the high activity DPEase were established. By establishing the composition of the additional medium (feeding solution) appropriate to the high concentration microbial cell culture, the conditions that the maximum activity was maintained in the high microbial cell concentration were established as follows.

5.1 Analysis of activity according to organic nitrogen source concentration

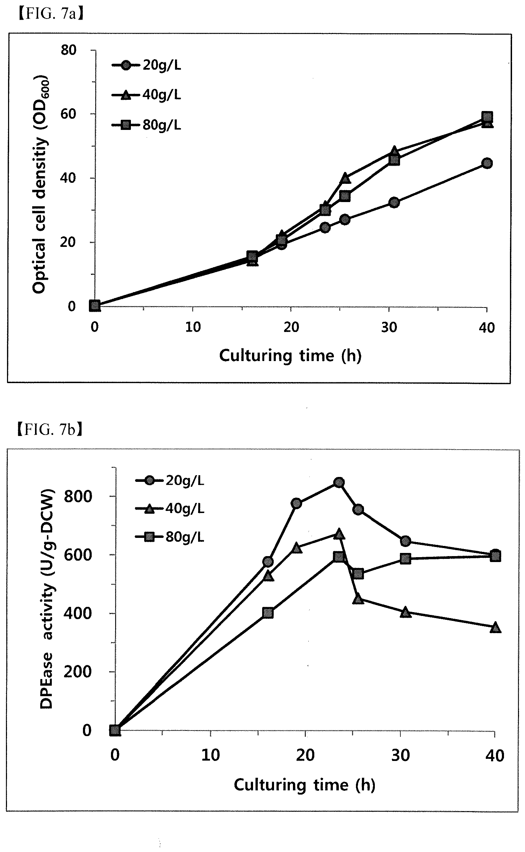

When the yeast extract is added to the saccharide supply solution as a complex organic nitrogen source, it may contribute to the microbial cell growth rate improvement and the enzyme activity increase. Thus, by controlling the yeast extract concentration in the saccharide supply solution to 20, 40, and 80 g/L, fermentation was progressed as Example 2. The pH-stat condition was progressed as same as Example 4. However, the composition of the additional medium (feeding solution) was psicose 400g/L and MnCl$_2$ 0.5mM, by using the yeast extract as a variable. In addition, the result obtained by comparing the enzyme activity (Unit/g-DCW) using the measurement method of activity conducted in Example 2 was shown in FIG. 7a and FIG. 7b and Table 5.

[Table 5]

| Culture time (hr) | 20g/L | 40g/L | 80g/L |
|---|---|---|---|
| 16 | 577 | 531 | 394 |
| 23.5 | 849 | 674 | 586 |
| 25.5 | 756 | 453 | 524 |
| 30.5 | 648 | 407 | 578 |
| 40 | 604 | 357 | 594 |

As shown in FIG. 7a and FIG. 7b, the DPEase activity was stably maintained as the concentration of yeast extract that was the complex organic nitrogen source in the additional medium (feeding solution) was increased. When the concentration of the organic nitrogen source was low, at the beginning of the pH-stat, the DPEase activity was the highest, but as the microbial cell concentration was raised, the DPEase activity was rapidly decreased. It was confirmed that it was required to establish the condition that the activity was stably maintained without decreasing the activity by the high concentration microbial cell concentration for psicose industrialization.

5.2 Analysis of activity according to manganese ion concentration

As the lower activity than the low concentration yeast extract in Example 5.1 was shown, for the high activity and high concentration culture, the activity according to the use of a metal ion, namely, manganese ion, which could act as a coenzyme increasing the activity of enzyme by increasing the binding capacity of fructose and DPEase, was analyzed.

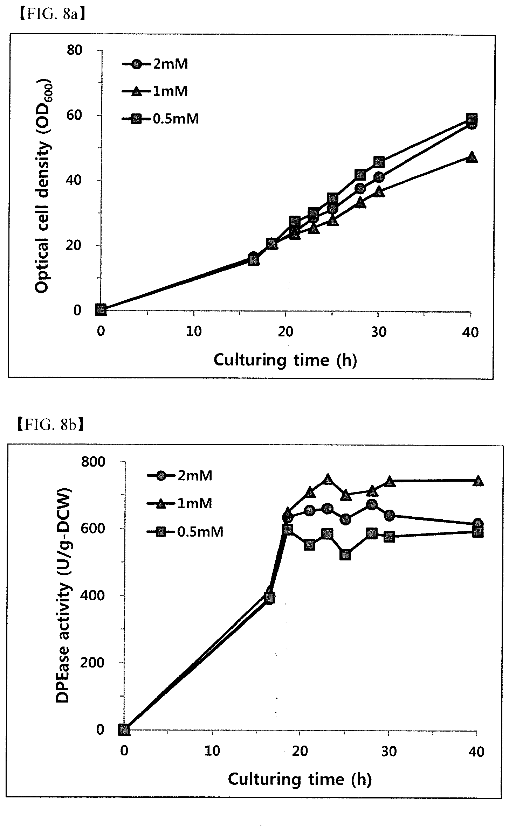

The psicose 400g/L and yeast extract 80g/L was in the saccharide supply solution, and the manganese concentration was adjusted to 0.5, 1.0, and 2.0mM, and fermentation was progressed as Example 2, and the pH-stat condition was progressed as same as Example 4. In addition, the result obtained by comparing the enzyme activity (Unit/g-DCW) using the measurement method of activity conducted in Example 2 was shown in FIG. 8a and FIG. 8b and Table 6.

[Table 6]

| Culture time (hr) | 0.5mM | 1.0mM | 2.0mM |
| --- | --- | --- | --- |
| 16.5 | 394 | 415 | 389 |
| 18.5 | 598 | 649 | 634 |
| 21 | 552 | 711 | 654 |
| 23 | 586 | 751 | 660 |
| 25 | 524 | 703 | 630 |
| 28 | 588 | 715 | 674 |
| 30 | 578 | 745 | 641 |
| 40 | 594 | 748 | 617 |

As shown in FIG. 8a and FIG. 8b, regardless of the manganese ion concentration in the additional medium (feeding solution), the activity was stably maintained all due to the appropriate C/N ratio, and the difference of the microbial cell growth rates was insignificant. It was confirmed that when using 1mM of manganese, the DPEase activity was over approximately 700 U/g after culture for 21 hours, and the highest DPEase activity compared to the low concentration 0.5mM or the high concentration 2mM was shown. Thus, it was confirmed that the manganese ion concentration so that the enzyme activity was maintained over approximately 700 U/g was 1 mM.

**EP 3 564 383 B1**

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Samyang Genex
263 Yonji-dong,
Chongro-gu,
Seoul 110-725,
Korea

RECEIPT IN THE CASE OF AN ORIGINAL
issued pursuant to Rule 7. 1 by the
INTERNATIONAL DEPOSITARY AUTHORITY
identified at the bottom of this page

| I. IDENTIFICATION OF THE MICROORGANISM | |
|---|---|
| Identification reference given by the DEPOSITOR : <br> *Microbacterium foliorum* SYG27B | Accession number given by the INTERNATIONAL DEPOSITARY AUTHORITY: <br> KCCM11774P |

**II. SCIENTIFIC DESCRIPTION AND/OR PROPOSED TAXONOMIC DESIGNATION**

The microorganism identified under I above was accompanied by:

☐ a scientific description

☐ a proposed taxonomic designation

(Mark with a cross where applicable)

**III. RECEIPT AND ACCEPTANCE**

This International Depositary Authority accepts the microorganism identified under I above, which was received by it on September. 24. 2015. (date of the original deposit)[1]

**IV. INTERNATIONAL DEPOSITARY AUTHORITY**

| Name : Korean Culture Center of Microorganisms <br><br> Address : Yurim B/D <br> 45, Hongjenae-2ga-gil <br> Seodaemun-gu <br> SEOUL 120-861 <br> Republic of Korea | Signature (s) of person (s) having the power to represent the International Depositary Authority or of authorized official (s) : <br><br> Date: September. 24. 2015. |
|---|---|

[1] Where Rule 6.4 (d) applies, such date is the date on which the status of international depositary authority was acquired; where a deposit made outside the Budapest Treaty after the acquisition of the status of international depositary authority is converted into a deposit under the Budapest Treaty, such date is the date on which the microorganism was received by the international depositary authority.

Form BP/4          Sole page

한국미생물보존센터

KOREAN CULTURE CENTER OF MICROORGANISMS

Yoolim Bldg., 45, Hongjenae 2ga-gil, Seodaemun-gu, Seoul, 120-861, Korea Tel: 82-2-391-0950, 396-0950 Fax: 82-2-392-2859

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

⌐　　　　　　　　　　　　　　　　　┐
To.　Samyang Genex
　　　263 Yonji-dong,
　　　Chongro-gu,　　　　　　　　　　RECEIPT IN THE CASE OF AN ORIGINAL
　　　Seoul 110-725,　　　　　　　　issued pursuant to Rule 7.1 by the
　　　　　　　　　　　　　　　　　INTERNATIONAL DEPOSITARY AUTHORITY
　　　Korea　　　　　　　　　　　　identified at the bottom of this page
∟　　　　　　　　　　　　　　　　　⌟

| I . IDENTIFICATION OF THE MICROORGANISM | |
|---|---|
| Identification reference given by the DEPOSITOR : *Ensifer adhaerens* SYG29 | Accession number given by the INTERNATIONAL DEPOSITARY AUTHORITY: KCCM11405P |

| II. SCIENTIFIC DESCRIPTION AND/OR PROPOSED TAXONOMIC DESIGNATION |
|---|
| The microorganism identified under I above was accompanied by: <br> ☐ a scientific description <br> ☐ a proposed taxonomic designation <br> (Mark with a cross where applicable) |

| III. RECEIPT AND ACCEPTANCE |
|---|
| This International Depositary Authority accepts the microorganism identified under I above, which was received by it on March. 29. 2013.　(date of the original deposit)[1] |

| IV. INTERNATIONAL DEPOSITARY AUTHORITY | |
|---|---|
| Name : Korean Culture Center of Microorganisms <br><br> Address : 361-221, Yurim B/D <br> Hongje-1-dong <br> Seodaemun-gu <br> SEOUL 120-091 <br> Republic of Korea | Signature(s) of person(s) having the power to represent the International Depositary Authority or of authorized official(s): <br><br> Date: March. 29. 2013. |

[1]　Where Rule 6.4(d) applies, such date is the date on which the status of international depositary authority was acquired; where a deposit made outside the Budapest Treaty after the acquisition of the status of international depositary authority is converted into a deposit under the Budapest Treaty, such date is the date on which the microorganism was received by the international depositary authority.

Form BP/4　　　　　　　　　　　　　　　　　　　　　　　　　　　　Sole page

**Claims**

1. A method for producing psicose, comprising

    a step of preparing an initial culture medium in which the ratio of the content (g/L) of carbon source to the content (g/L) of organic nitrogen source (C/N ratio) is 10/2 to 1/1, wherein the carbon source of the initial culture medium is at least one selected from the group consisting of psicose and fructose, and wherein the organic nitrogen source is at least one selected from the group consisting of yeast extract, corn steep liquor, soytone, peptone, soybean, soybean meal, cottonseed meal, molasses, casein, beef extract, malt extract and tryptone;
    a step of culturing a microorganism capable of producing psicose epimerase selected from the group consisting of Microbacterium foliorum, Microbacterium oxydans, Microbacterium phyllosphaerae and Ensifer adhaerens, in the initial culture medium which is performed in batch, in fed-batch or continuously; and
    a step of converting fructose into psicose, comprising reacting the cultured microorganism, a solution of the cultured microorganism, a lysate of the microorganism comprising psicose epimerase or a solution of the cultured microorganism, with fructose as a substrate, wherein the step of culturing is performed in a medium comprising 0.001 to 5 g/L of psicose as an inducer for increasing enzyme activity or expression of the microorganism under the condition that a pH variation value $\Delta$pH of the medium is 0.05 to 0.5, wherein, in the step of culturing, an additional medium comprising psicose as a carbon source in which additional medium a C/N ratio is 20/1 to 1/1, is supplied intermittently or continuously so that the carbon source concentration of the medium is maintained at 0.001 g/L to 5 g/L, wherein an f-value defined as an increase in carbon source concentration (g/L) in a culture solution when a carbon source is added once in the additional medium is 0.2 to 0.5.

2. The method for producing psicose according to claim 1, wherein metal ions are present at a concentration of 0.1 mM to 5 mM in the initial culture medium, or are added as an additional medium at a concentration of 0.1 mM to 5 mM to the initial culture medium.

3. The method for producing psicose according to claim 2, further comprising a step of supplying the additional medium comprising one or more of metal ions selected from the group consisting of manganese, cobalt, calcium, magnesium, nickel, iron and aluminum.

4. The method for producing psicose according to claim 1, wherein the supplying an additional medium is performed in a pH stat feeding method, wherein the pH stat feeding method supplies an additional medium comprising a carbon source so that a pH variation value $\Delta$pH in a culture solution is 0.05 to 0.5, and the carbon source concentration (g/L) in a culture solution is maintained at 0.001 g/L to 5 g/L.

5. The method for producing psicose according to claim 1, wherein the microorganism is a non-genetically modified microorganism comprising an inherent gene encoding psicose epimerase.

6. A method for increasing psicose epimerase activity or expression of a microorganism expressing a psicose epimerase, comprising a step of culturing a microorganism capable of producing psicose epimerase selected from the group consisting of Microbacterium foliorum, Microbacterium oxydans, Microbacterium phyllosphaerae and Ensifer adhaerens, in a medium comprising at least one selected from the group consisting of psicose and fructose as carbon source, and psicose as an inducer, wherein in said composition a ratio of the content (g/L) of carbon source to the content (g/L) of organic nitrogen source (C/N ratio) of the medium is 10/2 to 1/1, and wherein the organic nitrogen source is at least one selected from the group consisting of yeast extract, corn steep liquor, soytone, peptone, soybean, soybean meal, cottonseed meal, molasses, casein, beef extract, malt extract and tryptone, wherein the microorganism is cultured in a range of the inducer concentration (g/L) of 0.001 g/L to 5 g/L, and wherein the microorganism is cultured under the condition in which a pH variation value $\Delta$pH is 0.05 to 0.5, wherein, in the step of culturing, an additional medium comprising psicose as a carbon source in which additional medium a C/N ratio is 20/1 to 1/1, is supplied intermittently of continuously so that the carbon source concentration of the medium is maintained at 0.001 g/L to 5 g/L, wherein an f-value defined as an increase in carbon source concentration (g/L) in a culture solution when a carbon source is added once in the additional medium is 0.2 to 0.5.

7. The method according to claim 6, wherein the microorganism is cultured by adding an additional medium comprising the inducer intermittently or continuously.

8. The method according to claim 6, wherein the microorganism is a non-genetically modified microorganism comprising an inherent gene encoding psicose epimerase.

9. The method according to claim 6, wherein the step of culturing a microorganism is performed in a medium further comprising one or more of metal ions selected from the group consisting of manganese, cobalt, calcium, magnesium, nickel, iron and aluminum.

**Patentansprüche**

1. Verfahren zur Herstellung von Psicose, umfassend

   einen Schritt des Herstellens eines Ausgangskulturmediums, in dem das Verhältnis des Gehalts (g/L) an Kohlenstoffquelle zu dem Gehalt (g/L) an organischer Stickstoffquelle (C/N-Verhältnis) 10/2 bis 1/1 beträgt, wobei die Kohlenstoffquelle des Ausgangskulturmediums mindestens eine ist, die aus der Gruppe ausgewählt ist, die aus Psicose und Fructose besteht, und wobei die organische Stickstoffquelle mindestens eine ist, die aus der Gruppe ausgewählt ist, die aus Hefeextrakt, Maisquellwasser, Soyton, Pepton, Sojabohnen, Sojabohnenmehl, Baumwollsamenmehl, Melasse, Kasein, Rindfleischextrakt, Malzextrakt und Trypton besteht;
   einen Schritt des Kultivierens eines Mikroorganismus, der in der Lage ist, Psicoseepimerase zu produzieren, ausgewählt aus der Gruppe, die aus Microbacterium foliorum, Microbacterium oxydans, Microbacterium phyllosphaerae und Ensifer adhaerens besteht, in dem Ausgangskulturmedium, der als Batchprozess, Fed-Batch-Prozess oder kontinuierlich durchgeführt wird; und
   einen Schritt des Umwandelns von Fructose in Psicose, der ein Umsetzen des kultivierten Mikroorganismus, einer Lösung des kultivierten Mikroorganismus, eines Lysats des Mikroorganismus, das Psicoseepimerase oder eine Lösung des kultivierten Mikroorganismus umfasst, mit Fructose als Substrat umfasst, wobei der Schritt des Kultivierens in einem Medium, das 0,001 bis 5 g/L Psicose als einen Induktor zur Erhöhung der Enzymaktivität oder Expression des Mikroorganismus umfasst, unter der Bedingung, dass ein pH-Variationswert $\Delta$pH des Mediums 0,05 bis 0,5 beträgt, durchgeführt wird, wobei in dem Schritt des Kultivierens ein zusätzliches Medium, das Psicose als Kohlenstoffquelle umfasst, wobei ein C/N-Verhältnis in dem zusätzlichen Medium 20/1 bis 1/1 beträgt, periodisch oder kontinuierlich so zugeführt wird, dass die Kohlenstoffquellenkonzentration des Mediums bei 0,001 g/L bis 5 g/L gehalten wird, wobei ein f-Wert, der als eine Erhöhung der Kohlenstoffquellenkonzentration (g/L) in einer Kulturlösung, wenn eine Kohlenstoffquelle einmal in dem zusätzlichen Medium zugegeben wird, definiert ist, 0,2 bis 0,5 beträgt.

2. Verfahren zur Herstellung von Psicose nach Anspruch 1, wobei Metallionen in einer Konzentration von 0,1 mM bis 5 mM im Ausgangskulturmedium vorhanden sind oder als ein zusätzliches Medium bei einer Konzentration von 0,1 mM bis 5 mM dem Ausgangskulturmedium zugesetzt werden.

3. Verfahren zur Herstellung von Psicose nach Anspruch 2, ferner umfassend einen Schritt des Zuführens des zusätzlichen Mediums, das ein oder mehrere Metallionen, die aus der Gruppe ausgewählt sind, die aus Mangan, Kobalt, Calcium, Magnesium, Nickel, Eisen und Aluminium besteht, umfasst.

4. Verfahren zur Herstellung von Psicose nach Anspruch 1, wobei das Zuführen eines zusätzlichen Mediums in Form eines pH-Stat-Beschickungsverfahrens durchgeführt wird, wobei das pH-Stat-Beschickungsverfahren ein zusätzliches Medium, das eine Kohlenstoffquelle umfasst, so zuführt, dass ein pH-Variationswert $\Delta$pH in einer Kulturlösung 0,05 bis 0,5 beträgt und die Kohlenstoffquellenkonzentration (g/L) in einer Kulturlösung bei 0,001 g/L bis 5 g/L gehalten wird.

5. Verfahren zur Herstellung von Psicose nach Anspruch 1, wobei der Mikroorganismus ein nicht genetisch modifizierter Mikroorganismus ist, der ein eigenes Gen, das für Psicoseepimerase kodiert, umfasst.

6. Verfahren zur Erhöhung der Psicoseepimeraseaktivität oder Expression eines Mikroorganismus, der Psicoseepimerase exprimiert, umfassend einen Schritt des Kultivierens eines Mikroorganismus, der in der Lage ist, Psicoseepimerase zu produzieren, wobei der Mikroorganismus aus der Gruppe ausgewählt ist, die aus Microbacterium foliorum, Microbacterium oxydans, Microbacterium phyllosphaerae und Ensifer adhaerens besteht, in einem Medium, das mindestens einen Bestandteil, der aus der Gruppe ausgewählt ist, die aus Psicose und Fruktose besteht, als Kohlenstoffquelle und Psicose als einen Induktor umfasst, wobei in der Zusammensetzung ein Verhältnis des Gehalts (g/L) an Kohlenstoffquelle zu dem Gehalt (g/L) an organischer Stickstoffquelle (C/N-Verhältnis) des Mediums 10/2 bis 1/1 beträgt, und wobei die organische Stickstoffquelle mindestens eine ist, die aus der Gruppe, die aus Hefeextrakt, Maisquellwasser, Soyton, Pepton, Sojabohnen, Sojabohnenmehl, Baumwollsamenmehl, Melasse, Kasein, Rindfleischextrakt, Malzextrakt und Trypton besteht, ausgewählt ist, wobei der Mikroorganismus in einem Bereich der Induktorkonzentration (g/L) von 0,001 g/L bis 5 g/L kultiviert wird, und wobei der Mikroorganismus unter der Bedingung, bei der ein pH-Variationswert ΔpH des Mediums 0,05 bis 0,5 beträgt, kultiviert wird, wobei in dem Schritt des Kultivierens ein zusätzliches Medium, das Psicose als eine Kohlenstoffquelle umfasst, wobei ein C/N-Verhältnis in dem zusätzlichen Medium 20/1 bis 1/1 beträgt, periodisch oder kontinuierlich so zugeführt wird, dass die Kohlenstoffquellenkonzentration des Mediums bei 0,001 g/L bis 5 g/L gehalten wird, wobei ein f-Wert, der als eine Erhöhung der Kohlenstoffquellenkonzentration (g/L) in einer Kulturlösung, wenn eine Kohlenstoffquelle einmal in dem zusätzlichen Medium zugegeben wird, definiert ist, 0,2 bis 0,5 beträgt.

7. Verfahren nach Anspruch 6, wobei der Mikroorganismus durch periodisches oder kontinuierliches Hinzufügen eines zusätzlichen Mediums, das den Induktor umfasst, kultiviert wird.

8. Verfahren nach Anspruch 6, wobei der Mikroorganismus ein nicht genetisch modifizierter Mikroorganismus ist, der ein eigenes Gen, das für Psicoseepimerase kodiert, umfasst.

9. Verfahren nach Anspruch 6, wobei der Schritt des Kultivierens eines Mikroorganismus in einem Medium durchgeführt wird, das ferner ein oder mehrere Metallionen, die aus der Gruppe ausgewählt sind, die aus Mangan, Kobalt, Calcium, Magnesium, Nickel, Eisen und Aluminium besteht, umfasst.

**Revendications**

1. Procédé de production de psicose, comprenant

   une étape de préparation d'un milieu de culture initial dans lequel le rapport de la teneur (g/L) en source de carbone sur la teneur (g/L) en source d'azote organique (rapport C/N) est de 10/2 à 1/1, dans lequel la source de carbone du milieu de culture initial est au moins une sélectionnée dans le groupe constitué par le psicose et le fructose, et dans lequel la source d'azote organique est au moins une sélectionnée dans le groupe constitué par un extrait de levure, le liqueur de trempage du maïs, le soytone, la peptone, les graines de soja, la farine de soja, la farine de graines de coton, la mélasse, la caséine, un extrait de bœuf, un extrait de malt et la tryptone ; une étape de culture d'un micro-organisme capable de produire une psicose épimérase sélectionnée dans le groupe constitué par Microbacterium foliorum, Microbacterium oxydans, Microbacterium phyllosphaerae et Ensifer adhaerens, dans le milieu de culture initial qui est réalisée par lot, en semi-discontinu ou en continu ; et une étape de conversion du fructose en psicose, comprenant la mise en réaction du micro-organisme cultivé, d'une solution du micro-organisme cultivé, d'un lysat du micro-organisme comprenant une psicose épimérase ou une solution du micro-organisme cultivé, avec du fructose en tant que substrat, dans lequel l'étape de culture est réalisée dans un milieu comprenant 0,001 à 5 g/L de psicose en tant qu'inducteur pour augmenter l'activité enzymatique ou l'expression du micro-organisme dans la condition telle qu'une valeur de variation de pH ΔpH du milieu est de 0,05 à 0,5, dans lequel, à l'étape de culture, un milieu supplémentaire comprenant du psicose en tant que source de carbone, un rapport C/N étant de 20/1 à 1/1 dans ledit milieu supplémentaire, est alimenté de manière intermittente ou continue de telle sorte que la concentration en source de carbone du milieu est maintenue à 0,001 g/L à 5 g/L, dans lequel une valeur f définie comme une augmentation de la concentration en source de carbone (g/L) dans une solution de culture lorsqu'une source de carbone est ajoutée une fois dans le milieu supplémentaire est de 0,2 à 0,5.

2. Procédé de production de psicose selon la revendication 1, dans lequel des ions métalliques sont présents en une concentration de 0,1 mM à 5 mM dans le milieu de culture initial, ou sont ajoutés en tant que milieu supplémentaire en une concentration de 0,1 mM à 5 mM au milieu de culture initial.

3. Procédé de production de psicose selon la revendication 2, comprenant en outre une étape de fourniture du milieu supplémentaire comprenant un ou plusieurs d'ions métalliques sélectionnés dans le groupe constitué par le manganèse, le cobalt, le calcium, le magnésium, le nickel, le fer et l'aluminium.

4. Procédé de production de psicose selon la revendication 1, dans lequel la fourniture d'un milieu supplémentaire est réalisée dans un procédé de fourniture de pH-stat, dans lequel le procédé de fourniture de pH-stat fournit un milieu supplémentaire comprenant une source de carbone de telle sorte qu'une valeur de variation du pH ΔpH dans une solution de culture est de 0,05 à 0,5, et la concentration en source de carbone (g/L) dans une solution de culture est maintenue à 0,001 g/L à 5 g/L.

**5.** Procédé de production de psicose selon la revendication 1, dans lequel le microorganisme est un microorganisme non génétiquement modifié comprenant un gène inhérent codant pour une psicose épimérase.

**6.** Procédé d'augmentation de l'activité de la psicose épimérase ou de l'expression d'un microorganisme exprimant une psicose épimérase, comprenant une étape de culture d'un micro-organisme capable de produire une psicose épimérase sélectionné dans le groupe constitué par Microbacterium foliorum, Microbacterium oxydans, Microbacterium phyllosphaerae et Ensifer adhaerens, dans un milieu comprenant au moins l'un sélectionné dans le groupe constitué par le psicose et le fructose en tant que source de carbone, et le psicose en tant qu'inducteur, dans lequel dans ladite composition un rapport de la teneur (g/L) en source de carbone sur la teneur (g/L) en source d'azote organique (rapport C/N) du milieu est de 10/2 à 1/1, et dans lequel la source d'azote organique est au moins une sélectionnée dans le groupe constitué par un extrait de levure, le liqueur de trempage du maïs, le soytone, la peptone, le soja, la farine de soja, la farine de graines de coton, la mélasse, la caséine, un extrait de bœuf, un extrait de malt et la tryptone, dans lequel le microorganisme est cultivé dans une plage de concentration en inducteur (g/L) de 0,001 g/L à 5 g/L, et dans lequel le microorganisme est cultivé dans la condition telle qu'une valeur de variation de pH ∆pH est de 0,05 à 0,5, dans lequel, à l'étape de culture, un milieu supplémentaire comprenant du psicose en tant que source de carbone, un rapport C/N étant de 20/1 à 1/1 dans ledit milieu supplémentaire, est alimenté de manière intermittente ou continue de telle sorte que la concentration en source de carbone du milieu est maintenue à 0,001 g/L à 5 g/L, dans lequel une valeur f définie comme une augmentation de la concentration en source de carbone (g/L) dans une solution de culture lorsqu'une source de carbone est ajoutée une fois dans le milieu supplémentaire est de 0,2 à 0,5.

**7.** Procédé selon la revendication 6, dans lequel le microorganisme est cultivé par ajout d'un milieu supplémentaire comprenant l'inducteur de manière intermittente ou continue.

**8.** Procédé selon la revendication 6, dans lequel le microorganisme est un microorganisme non génétiquement modifié comprenant un gène inhérent codant pour une psicose épimérase.

**9.** Procédé selon la revendication 6, dans lequel l'étape de culture d'un microorganisme est réalisée dans un milieu comprenant en outre un ou plusieurs d'ions métalliques sélectionnés dans le groupe constitué par le manganèse, le cobalt, le calcium, le magnésium, le nickel, le fer et l'aluminium.

【FIG. 1】

on/off control

pulse control

[FIG. 2a]

【FIG. 2b】

DPEase activity (U/g-DCW)

Cell density (OD600)

【FIG.3a】

【FIG.3b】

【FIG.3c】

【FIG. 4a】

【FIG. 4b】

【FIG. 5a】

【FIG. 5b】

【FIG. 5c】

【FIG. 6a】

【FIG. 6b】

【FIG. 7a】

【FIG. 7b】

【FIG. 8a】

【FIG. 8b】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2730652 A1
- WO 2016053035 A1
- US 2017298400 A1
- WO 2016201110 A1
- EP 3088515 A1
- KR 1020140122043